(19)

Europäisches Patentamt

European Patent Office

Office européen des brevets

(11) **EP 4 535 195 A1**

(12) **EUROPEAN PATENT APPLICATION**
published in accordance with Art. 153(4) EPC

(43) Date of publication:
**09.04.2025 Bulletin 2025/15**

(21) Application number: **23811774.1**

(22) Date of filing: **22.05.2023**

(51) International Patent Classification (IPC):
***G06F 16/906*** (2019.01)   ***G06Q 10/04*** (2023.01)

(52) Cooperative Patent Classification (CPC):
**G06F 16/906; G06Q 10/04; G06Q 50/10**

(86) International application number:
**PCT/JP2023/018957**

(87) International publication number:
**WO 2023/228902 (30.11.2023 Gazette 2023/48)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **26.05.2022   JP 2022086247**

(71) Applicants:
• **DAIKIN INDUSTRIES, LTD.**
**Osaka-shi, Osaka 530-0001 (JP)**
• **The University of Tokyo**
**Bunkyo-ku, Tokyo 113-8654 (JP)**

(72) Inventors:
• **MIYAMOTO, Yumi**
**Osaka-Shi, Osaka 530-0001 (JP)**
• **SHIMIZU, Aori**
**Osaka-Shi, Osaka 530-0001 (JP)**
• **MIURA, Takahiro**
**Tokyo 113-8654 (JP)**
• **ASATANI, Kimitaka**
**Tokyo 113-8654 (JP)**
• **SAKATA, Ichiro**
**Tokyo 113-8654 (JP)**

(74) Representative: **Goddar, Heinz J.**
**Boehmert & Boehmert**
**Anwaltspartnerschaft mbB**
**Pettenkoferstrasse 22**
**80336 München (DE)**

(54) **ASSESSMENT ASSISTANCE DEVICE, ASSESSMENT METHOD, AND PROGRAM**

(57)   The tendency of classification related to the hazard of a chemical substance can be evaluated or predicted. A control unit of an evaluation support apparatus outputs information for evaluating or predicting the tendency of classification regarding the hazard of a chemical substance, based on the relatedness of a plurality of documents.

FIG.1

EP 4 535 195 A1

**Description**

Technical Field

The present disclosure relates to an evaluation support apparatus, an evaluation method, and a program.

Background Art

There is a technique for predicting the toxicity of a chemical substance based on the structural characteristics of the chemical substance. For example, Patent Document 1 discloses an invention for vectorizing the structure of a chemical substance and calculating a toxicity prediction score by using a learned classifier.

Citation List

Patent Document

[Patent document 1] WO 2018/049376

Summary of Invention

Technical Problem

However, in the regulation of chemical substances, standards may change due to external factors such as social factors in addition to the toxicity of chemical substances themselves. Therefore, it is difficult to evaluate or predict the hazard of chemical substances only from the structural characteristics of the chemical substances.
The present disclosure makes it possible to evaluate or predict the tendency of classification related to the hazard of chemical substances.

Solution to Problem

An evaluation support apparatus according to a first aspect of the present disclosure includes:

a control unit, wherein
the control unit outputs information for evaluating or predicting a tendency of classification regarding a hazard of a chemical substance, based on relatedness of a plurality of documents.

According to the first aspect of the present disclosure, the tendency of classification related to the toxicity of a chemical substance can be evaluated or predicted.
A second aspect of the present disclosure is the evaluation support apparatus according to the first aspect,, wherein the control unit performs statistical processing of information classifying the plurality of documents for each property of the chemical substance, to thereby output the information for evaluating or predicting the tendency of the classification related to the hazard of the chemical substance.
A third aspect of the present disclosure is the evaluation support apparatus according to the second aspect, wherein the control unit classifies the plurality of documents based on whether or not the property of the chemical substance is described.
A fourth aspect of the present disclosure is the evaluation support apparatus according to the third aspect, wherein the control unit performs the statistical processing of the information classifying the plurality of documents based on characteristic information of the chemical substance described in the document.
A fifth aspect of the present disclosure is the evaluation support apparatus according to the fourth aspect, wherein the control unit evaluates or predicts the tendency of the classification related to the hazard of the chemical substance, from the output information.
A sixth aspect of the present disclosure is the evaluation support apparatus according to the fifth aspect, wherein the control unit evaluates or predicts the tendency of the chemical substances having similar characteristic information, from the characteristic information of the chemical substance described in the document.
A seventh aspect of the present disclosure is the evaluation support apparatus according to the second to the sixth aspect, wherein the property of the chemical substance includes at least one of toxicity, bioaccumulation, recalcitrance, regional distribution, flammability, or greenhouse effect of the chemical substance.
An eighth aspect of the present disclosure is the evaluation support apparatus according to the second to the sixth aspect, wherein the property of the chemical substance includes information that lowers an applicability to the classification related to the hazard of the chemical substance.

A ninth aspect of the present disclosure is the evaluation support apparatus according to the eighth aspect, wherein the information lowering the applicability includes at least one of a processing method or a decomposition method of the chemical substance.

A tenth aspect of the present disclosure is the evaluation support apparatus according to the second aspect, wherein the control unit classifies the plurality of documents based on a distributed expression in which the documents highly related to each other are arranged close to each other.

An eleventh aspect of the present disclosure is the evaluation support apparatus according to the tenth aspect, wherein the control unit

attaches, to a document among the plurality of documents, document information indicating whether or not the property of the chemical substance is described, and

classifies the document in which the property of the chemical substance is described, based on the document information.

A twelfth aspect of the present disclosure is the evaluation support apparatus according to the second to eleventh aspects, wherein the control unit classifies the plurality of documents based on a citation relationship between the plurality of documents.

A thirteenth aspect of the present disclosure is the evaluation support apparatus according to the twelfth aspect, wherein the document is an academic paper.

A fourteenth aspect of the present disclosure is the evaluation support apparatus according to the second to thirteenth aspects, wherein the control unit classifies the plurality of documents based on a result of natural language processing of contents described in the document.

An evaluation method according to the fifteenth aspect of the present disclosure includes:

a procedure of evaluating or predicting a tendency of classification related to a hazard of a chemical substance, based on relatedness of a plurality of documents.

A program according to a sixteenth aspect of the present disclosure causes a control unit included in an evaluation support apparatus to execute:

a procedure of outputting information for evaluating or predicting a tendency of classification related to a hazard of a chemical substance, based on relatedness of a plurality of documents.

Brief Description of Drawings

[FIG. 1] FIG. 1 is a block diagram illustrating an example of the system configuration of the evaluation support apparatus.

[FIG. 2] FIG. 2 is a block diagram illustrating an example of the hardware configuration of the evaluation support apparatus.

[FIG. 3] FIG. 3 is a block diagram illustrating an example of the functional configuration of the evaluation support apparatus.

[FIG. 4] FIG. 4 is a flowchart illustrating the processing procedure of the evaluation support apparatus.

[FIG. 5] FIG. 5 is a conceptual diagram illustrating an example of a boundary surface.

[FIG. 6] FIG. 6 is a conceptual diagram illustrating an example of output results.

[FIG. 7] FIG. 7 is a conceptual diagram illustrating an example of output results.

Description of Embodiments

Each embodiment will be described below with reference to the attached drawings. In the present specification and the drawings, elements having substantially the same functional configuration will be denoted by the same reference numerals, thereby omitting duplicate descriptions.

[Embodiment]

The present embodiment is an evaluation support apparatus which outputs information for evaluating or predicting the tendency of classification related to the hazard of chemical substances. The evaluation support apparatus of the present embodiment learns a classifier for each classification related to the hazard of chemical substances based on the relatedness of a plurality of documents collected about chemical substances, and classifies documents to be investigated. Further, the evaluation support apparatus of the present embodiment outputs information for evaluating or predicting the tendency of classification related to the hazard of chemical substances by statistically processing the information that classifies documents for each property of chemical substances.

The classification related to the hazard of chemical substances is a group in which chemical substances are classified based on the nature of the harm they cause to humans, organisms, or the environment. An example of the classification related to the hazard of a chemical substance is whether or not the chemical substance falls under the category of CMR (carcinogenic, mutagenic or toxic for reproduction) substances, PBT (persistence, bioaccumulation and toxicity) substances, vPvB (very persistent and very bioaccumulative) substances, etc., in the European REACH (registration, evaluation, authorisation and restriction of chemicals) regulation. The CMR substances are chemical substances that are designated as substances that affect human health. The PBT substances are chemical substances that have recalcitrance, bioaccumulation, or toxicity and are designated as substances that affect the environment. The vPvB substances are chemical substances that are designated as substances that have extremely high recalcitrance and bioaccumulation.

<System configuration>

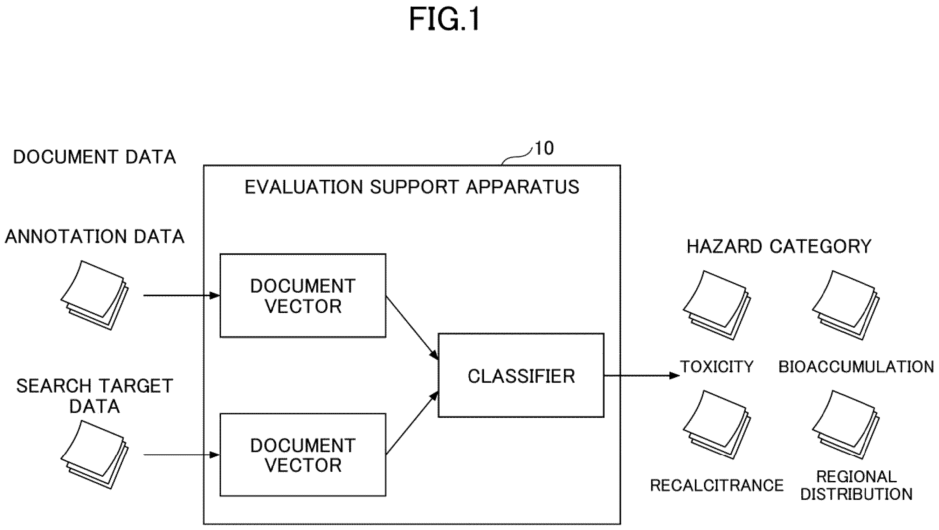

FIG. 1 is a block diagram illustrating an example of the system configuration of an evaluation support apparatus 10 in the present embodiment. As illustrated in FIG. 1, the evaluation support apparatus 10 inputs document data including annotation data and search target data. The evaluation support apparatus 10 converts each input document data into a document vector and learns a classifier for each category related to the hazard of chemical substances based on the relatedness of the document data. The evaluation support apparatus 10 classifies the input search target data into each category and outputs information for evaluating or predicting the tendency of classification related to the hazard of chemical substances based on the statistical information for each category.

The document data in the present embodiment is data representing documents related to chemical substances. An example of the document data is paper data representing the contents of academic papers related to chemical substances. The paper data can be collected by using a paper database or the like. As the paper database, for example, SCOPUS (registered trademark) can be used.

Another example of document data is patent publications related to chemical substances. The patent publications may be collected from publications issued by national patent offices, or a database containing publications issued by each of the national patent offices may be used.

The document data need not represent the entire document (for example, academic papers or patent specifications, etc.). The document data may represent a part of the document or a summary.

Characteristic information of the chemical substance described in the document is attached to the document data. One example of the characteristic information is identification information for identifying the chemical substance. Another example of the characteristic information is the fingerprint of the compound or information about the functional group or skeleton.

The identification information for identifying a chemical substance is, for example, a chemical compound name, a name based on IUPAC (International Union of Pure and Applied Chemistry) nomenclature, a notation based on SMILES notation, an InChI (International Chemical Identifier) Key, or a structural formula. The identification information is not limited to these, but any information that can identify a chemical substance can be used.

The characteristic information of a chemical substance described in a document sometimes contains a lot of noise such as notation variations. A notation variation indicates that different characteristic information is given to the same substance. Therefore, it is good to eliminate notation variations by using a chemical substance database, etc., for the characteristic information added to the document data. An example of a chemical substance database is the Japan Chemical Substance Dictionary.

Annotation data is document data to which document information is added. The search target data is document data to which document information is not attached. Document information is information indicating whether or not the properties of chemical substances are described in the document data. The document information may be a truth value obtained by binary classification of the document data as to whether or not the document data corresponds to each property.

The relatedness of the documents is the relatedness based on the content described in the document data. The relatedness of the documents may be based on the properties of chemical substances, etc.

An example of the properties of chemical substances is information representing a category related to the hazard of chemical substances. The category related to the hazard of chemical substances includes, for example, at least one of toxicity, bioaccumulation, recalcitrance, regional distribution, flammability, or greenhouse effect of chemical substances. The category related to the hazard of chemical substances is not limited to these but may include other categories.

Another example of the property of a chemical substance is information that represents a category related to environmental technologies. The environmental technologies are, for example, methods of treating or decomposing a chemical substance. Appropriate treatment or decomposition of a chemical substance may reduce the hazard of the chemical substance. Therefore, the information related to environmental technologies is information that lowers the applicability to the category related to the hazard of the chemical substance. The properties of the chemical substance are not limited to these, but may include other properties.

The statistical information in the present embodiment is the aggregate result of the identification information of the chemical substance and the documents classified into the category related to the hazard. The statistical information may be the increase rate of the number of pieces of document data, the percentage of each category, their time series transition, etc. The statistical information may be the aggregate result based on the bibliographic information of the documents. The bibliographic information includes, for example, the year of publication, the issuing institution, the author, or the like. The statistical information may be aggregated based on the density of the network of institutions and authors.

An example of information for evaluating or predicting a classification related to the hazard of a chemical substance is information indicating the time series transition of the number of documents classified into each category for a chemical substance. By referring to such information, it is possible to identify the category in which the hazard of the chemical substance has been actively discussed recently.

Another example of information for evaluating or predicting a classification related to the hazard of a chemical substance is information indicating the list of chemical substances with the largest number of documents, among the documents classified into each category. By referring to such information, it is possible to identify the chemical substance that has been actively discussed recently in the hazard category.

<Hardware configuration>

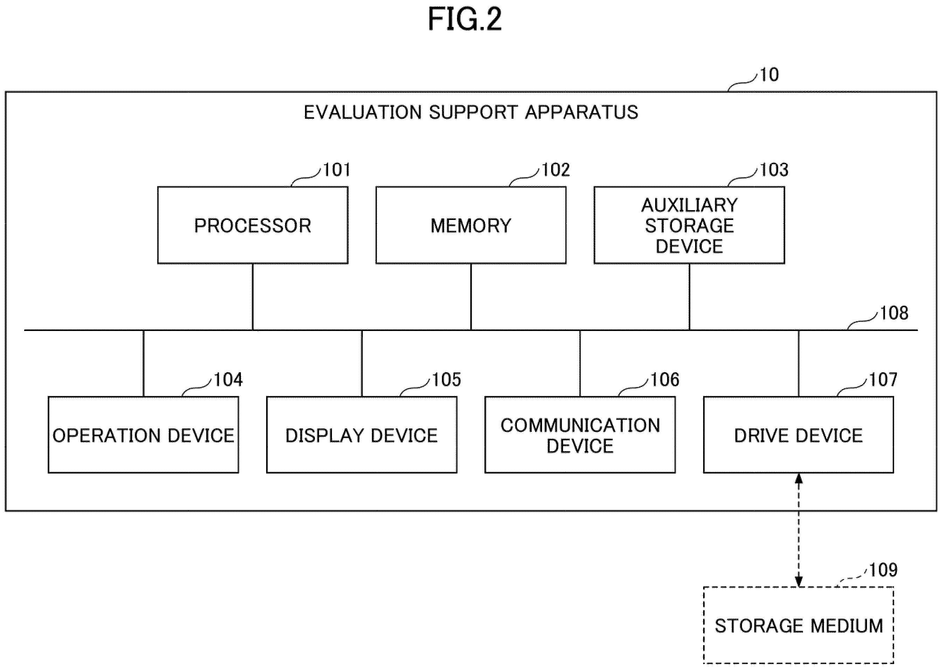

FIG. 2 is a block diagram illustrating an example of the hardware configuration of the evaluation support apparatus 10 in the present embodiment. As illustrated in FIG. 2, the evaluation support apparatus 10 includes a processor 101, a memory 102, an auxiliary storage device 103, an operation device 104, a display device 105, a communication device 106, and a drive device 107. The hardware of the evaluation support apparatus 10 is connected to each other via a bus 108.

The processor 101 includes various computing devices such as a CPU (Central Processing Unit). The processor 101 reads various programs installed in the auxiliary storage device 103 onto the memory 102 and executes the programs.

The memory 102 includes main storage devices such as a ROM (Read Only Memory) and a RAM (Random Access Memory). The processor 101 and the memory 102 form what is referred to as a computer (hereinafter also referred to as a "control unit"), and when the processor 101 executes various programs read onto the memory 102, the computer implements various functions.

The auxiliary storage device 103 stores various programs and various kinds of data used when the various programs are executed by the processor 101.

The operation device 104 is an operation device for the user of the evaluation support apparatus 10 to perform various operations. The display device 105 is a display device for displaying the processing results of various processes executed by the evaluation support apparatus 10.

The communication device 106 is a communication device for communicating with an external device via a network (not illustrated).

The drive device 107 is a device for setting a storage medium 109. The storage medium 109 includes a medium for storing information optically, electrically, or magnetically, such as a CD-ROM, a flexible disk, or a magneto-optical disk. The storage medium 109 may also include a semiconductor memory for storing information electrically, such as a ROM, a flash memory, or the like.

Various programs installed in the auxiliary storage device 103 are installed, for example, when the distributed storage medium 109 is set in the drive device 107 and various programs stored in the storage medium 109 are read by the drive device 107. Alternatively, various programs installed in the auxiliary storage device 103 may be installed by downloading them from the network via the communication device 106.

<Functional configuration>

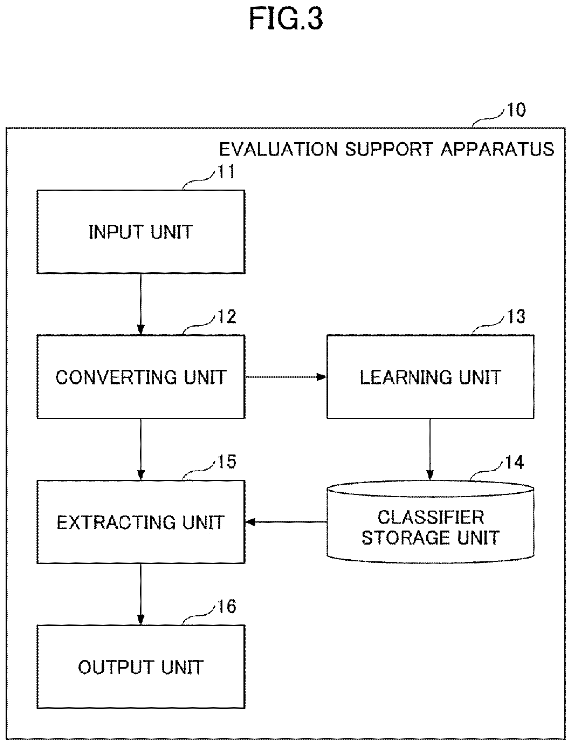

FIG. 3 is a block diagram illustrating an example of the functional configuration of the evaluation support apparatus in the present embodiment. As illustrated in FIG. 3, the evaluation support apparatus 10 in the present embodiment includes an input unit 11, a converting unit 12, a learning unit 13, a classifier storage unit 14, an extracting unit 15, and an output unit 16.

The classifier storage unit 14 is implemented by a memory 102 or an auxiliary storage device 103 illustrated in FIG. 2. The input unit 11, the converting unit 12, the learning unit 13, the extracting unit 15, and the output unit 16 are implemented by executing various programs read from the memory 102 by the processor 101 illustrated in FIG. 2.

The input unit 11 receives input of a plurality of pieces of document data. The document data includes a plurality of pieces of annotation data and a plurality of pieces of search target data.

The converting unit 12 generates a document vector for each piece of document data received by the input unit 11.

The learning unit 13 learns a classifier for each category related to the hazard based on the document vector generated by the converting unit 12.

The classifier storage unit 14 stores a classifier for each category learned by the learning unit 13.

The extracting unit 15 classifies the search target data received by the input unit 11 into categories by using a classifier stored in the classifier storage unit 14.

The output unit 16 outputs information for evaluating or predicting the hazard of chemical substances based on the statistical information for each category.

<Flow of the evaluation support method>

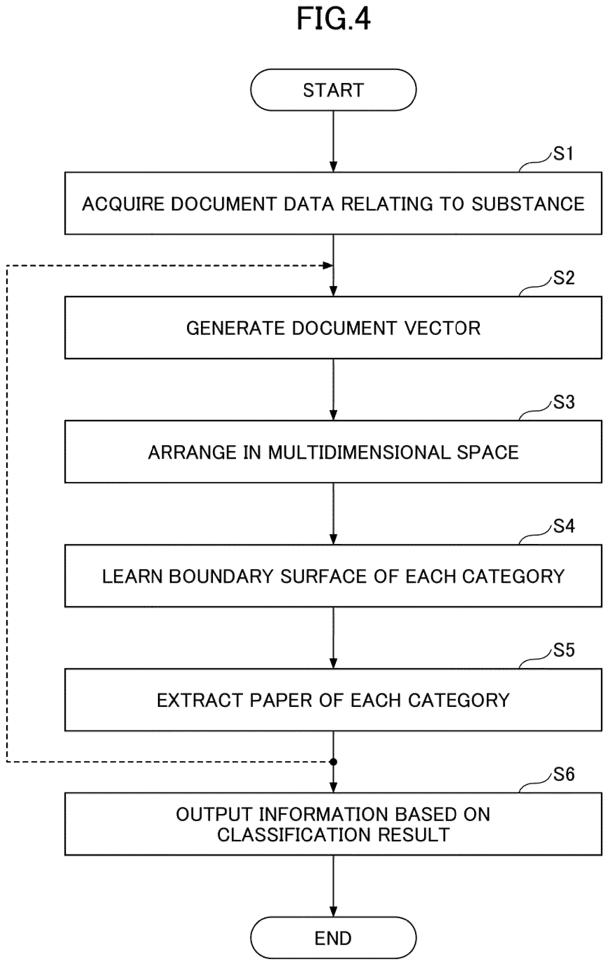

FIG. 4 is a flowchart illustrating an example of the flow of the evaluation support method in the present embodiment.

In step S1, the input unit 11 receives input of a plurality of pieces of document data. The document data includes a plurality of pieces of annotation data and a plurality of search target data. Next, the input unit 11 sends the received document data to the converting unit 12.

The number of pieces of annotation data may be any number that is greater than or equal to the number required for the learning unit 13 to learn the classifier. For example, the number of pieces of annotation data may be approximately 100 to 500 for each category. The number of pieces of search target data is not limited, but may be about 10,000, for example.

In step S2, the converting unit 12 receives the document data from the input unit 11. Next, the converting unit 12 generates document vectors for each piece of document data. Subsequently, the converting unit 12 sends the generated document vectors to the learning unit 13.

The document vector in the present embodiment consists of at least one of distributed expressions in a citation space or distributed expressions in a document space. Distributed expressions in a citation space are generated based on the citation relationship between pieces of document data. An example of distributed expressions in a citation space is LINE (Large-scale Information Network Embedding). Details about LINE are disclosed in Reference 1 below.

[Reference 1] Tang, J., Qu, M., Wang, M., Zhang, M., Yan, J., and Mei, Q, "Line: Large-scale information network embedding" in Proceedings of the 24th international conference on world wide web, pp. 1067-1077, 2015.

In LINE, there are distributed expressions based on first-order proximity and distributed expressions based on second-order proximity. In the present embodiment, both distributed expressions based on first-order proximity and distributed expressions based on second-order proximity can be used, but it is preferable to use distributed expressions based on second-order proximity.

A distributed expression based on first-order proximity is a probability expression that indicates whether pairs of nodes are linked or not. A distributed expression based on first-order proximity is expressed by equation (1). Note that $v$ represents a node, and u is a low-dimensional vector representation of $v$.

[Equation 1]

$$P_1\left(v_1, v_j\right) = \frac{1}{1 + exp\left(-\vec{u}_i^T \cdot \vec{u}_j\right)} \quad \cdots (1)$$

A distributed expression based on second-order proximity is a probability representation of whether a pair of nodes have a common link with another node. The distributed expression based on second-order proximity is expressed by equation (2). Note that v represents a node, u is a low-dimensional vector representation of $v$, and u' is a vector representation of context.

[Equation 2]

$$P_2\left(v_i \middle| v_j\right) = \frac{exp\left(-\vec{u}_i^T \cdot \vec{u}_j\right)}{\sum_{k=1}^{|V|} exp\left(\vec{u'}_k^T \cdot \vec{u}_i\right)} \quad \cdots (2)$$

The distributed expression in the document space is generated based on the contents of the document data. An example of the distributed expression in the document space is BERT (Bidirectional Encoder Representations from Transformers). More details about BERT are disclosed in Reference 2 below.

[Reference 2]

Beltagy, I., Lo, K., and Cohan, A., "SciBERT: A pretrained language model for scientific text", arXiv preprint, arXiv:1903.10676, 2019.

The converting unit 12 may generate the distributed expression in the citation space as a document vector, and generate the distributed expression in the document space as a document vector. The converting unit 12 may generate the distributed expression in the citation space and the distributed expression in the document space respectively, and the vector obtained by combining them may be a document vector.

In step S3, the learning unit 13 receives a plurality of document vectors corresponding to each piece of document data from the converting unit 12. Next, the learning unit 13 arranges each document vector in a multidimensional space. The multidimensional space in the present embodiment is configured so that document vectors having high relatedness with each other are arranged close to each other. At this time, all document vectors including annotation data and search target data are arranged in the multidimensional space.

In step S4, the learning unit 13 learns a classifier for each category in the multidimensional space in which the document vectors are arranged. The classifier in the present embodiment is a boundary surface dividing the multidimensional space into two spaces. The learning unit 13 learns the boundary surface of the relevant category so that among the document vectors arranged in the multidimensional space, the document vectors corresponding to the annotation data given the category to be learned is gathered in one space obtained by division by the boundary surface.

The boundary surface in the present embodiment is learned by logistic regression. However, the learning method of the boundary surface is not limited to logistic regression, and any learning method may be used as long as the classifier is capable of binary classification of multidimensional space.

The boundary surface in the present embodiment is learned for each category. For example, when a category including toxicity, bioaccumulation, recalcitrance, and regional distribution of chemical substances is used, the control unit learns the boundary surface related to toxicity, the boundary surface related to bioaccumulation, the boundary surface related to recalcitrance, and the boundary surface related to regional distribution, respectively.

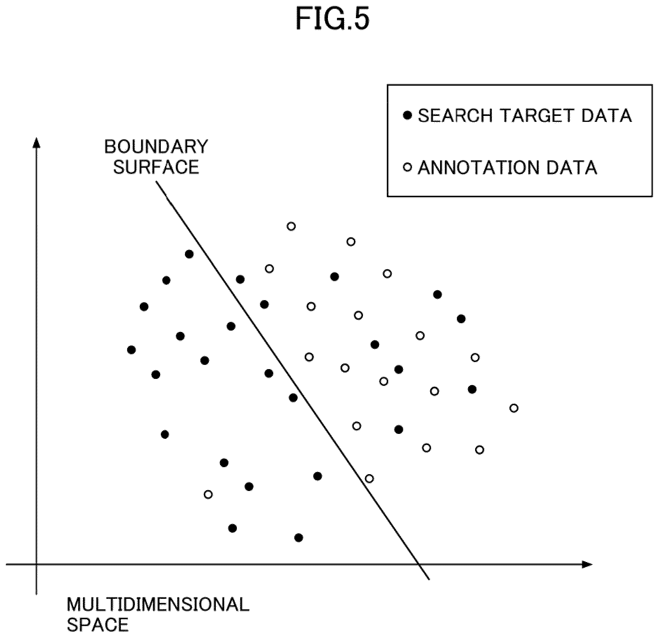

FIG. 5 is a conceptual diagram indicating an example of the boundary surface. In FIG. 5, white circles represent the arrangement of annotation data in a multidimensional space, and black circles represent the arrangement of search target data in a multidimensional space. As illustrated in FIG. 5, the boundary surface is learned so that annotation data is gathered as much as possible in one space obtained by division by the boundary surface.

The boundary surface for each category is re-learned each time the annotation data and the search target data are added. As described later, annotation data can be added by adding a classification result to the search target data. The learning unit 13 can re-learn the boundary surface after the annotation data is added.

In step S5, the extracting unit 15 reads the classifiers (boundary surface) for each category stored in the classifier storage unit 14. Next, the extracting unit 15 classifies the search target data by using the boundary surface for each category. The extracting unit 15 classifies each piece of search target data by determining whether or not it corresponds to the category for each category.

The extracting unit 15 adds the classification result for each category to the search target data. Next, the extracting unit 15 sends the search target data to which the classification result is added to the output unit 16.

The extracting unit 15 may learn the boundary surface for each category again by using the search target data given the classification result as annotation data. At this time, new search target data is collected and the document vectors are arranged in a multidimensional space. As a result, it is possible to increase the document data for each category related to the hazard and to evaluate the hazard of the chemical substance with more accuracy.

In step S6, the output unit 16 receives, from the extracting unit 15, the search target data to which the classification result is attached. Next, the output unit 16 generates statistical information for each category based on the classification result. The statistical information in the present embodiment is, for example, statistical information in which the number of cases is aggregated based on the identification information of the chemical substance and the category related to the hazard. The statistical information in the present embodiment may be aggregated based on the year of publication of the document, and the bibliographic information which is the publishing organization or the author.

Subsequently, the output unit 16 outputs information for evaluating or predicting the tendency of classification related to the hazard of the chemical substance based on the statistical information for each category. One example of the information to be output is information representing the transition of the number of documents classified into each category for a certain chemical substance. Another example of the information to be output is information representing a list of chemical substances having a large number of documents among the documents classified into each category.

The output unit 16 may evaluate or predict the tendency of classification related to the hazard of the chemical substance based on the information for evaluating or predicting the tendency of classification related to the hazard of the chemical substance and output the result. The evaluation result of the hazard is, for example, a score calculated according to a predetermined rule for each category of the hazard. The prediction result of the hazard is information representing a category that is highly likely to be discussed in the future for a certain chemical substance or information representing a chemical substance that is highly likely to be discussed in the future for a certain category.

The output unit 16 may evaluate or predict the tendency related to the hazard of chemical substances with similar characteristic information of the chemical substance described in the document data. For example, if the chemical substances have similar compound names and chemical formulas, the tendency of classification related to the hazard is also likely to be similar. Therefore, it is possible to obtain useful information by simultaneously evaluating or predicting chemical substances with similar characteristic information.

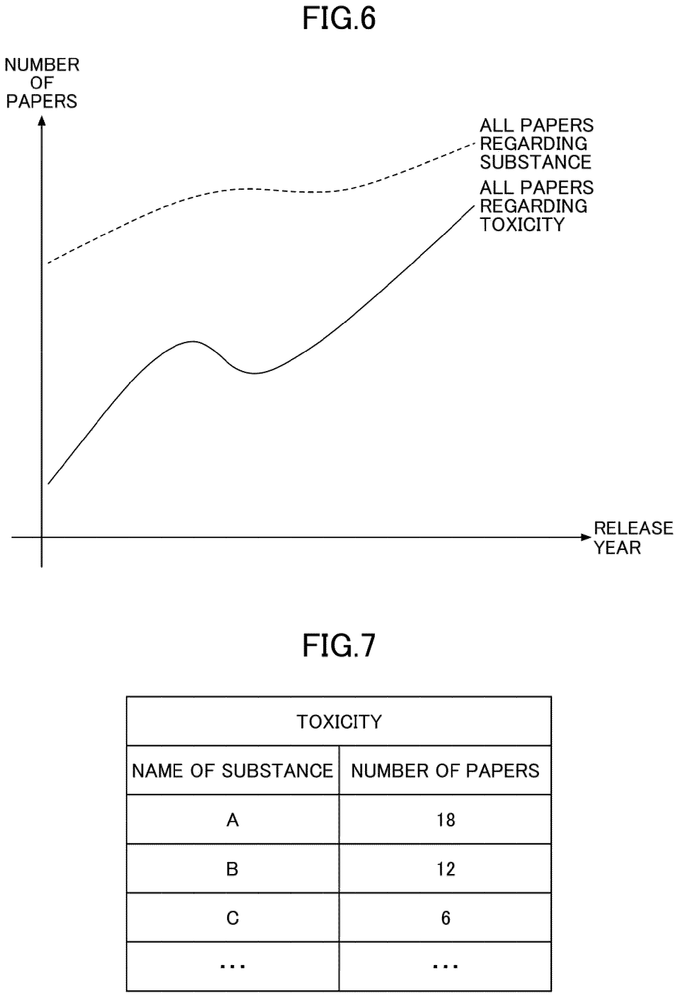

FIG. 6 is an example of output results indicating the transition of the number of documents classified into each category. As illustrated in FIG. 6, one example of output results outputs the transition of the number of all papers and the number of papers in each category for each publication year of a chemical substance in a form that can be compared. According to this output result, it is possible to identify, for example, that there has been a large number of discussions about the toxicity of a chemical substance recently.

FIG. 7 is an example of output results indicating a list of chemical substances with a large number of papers among the documents classified into each category. As illustrated in FIG. 7, another example of output results outputs a list of substances with a large number of papers in the category relating to the hazard (for example, toxicity) in descending order. According to this output result, it is possible to identify, for example, chemical substances with a large number of discussions about the toxicity of a chemical substance recently.

<Flow of evaluation method>

The user of the evaluation support apparatus 10 in the present embodiment can evaluate or predict the tendency of classification related to the hazard of chemical substances by using the evaluation support apparatus 10. The evaluation method using the evaluation support apparatus 10 will be described below.

The user of the evaluation support apparatus 10 inputs a plurality of pieces of document data to the evaluation support apparatus 10. The evaluation support apparatus 10 receives input of a plurality of pieces of document data (step S1 in FIG. 4). The evaluation support apparatus 10 executes steps S2 to S5 of the evaluation support method based on the received plurality of pieces of document data. Then, the evaluation support apparatus 10 outputs information for evaluating or predicting the tendency of classification related to the hazard of chemical substances (step S6 in FIG. 4).

The user of the evaluation support apparatus 10 evaluates or predicts the tendency of classification related to the hazard of chemical substances based on the information output from the evaluation support apparatus 10. For example, the user of the evaluation support apparatus 10 refers to the statistical information of the classification related to the hazard of a specific chemical substance, so that the user can identify the chemical substance whose hazard is actively discussed or the classification of the hazard that is actively discussed with respect to a specific chemical substance.

<Summary>

As described above, according to each embodiment of the present disclosure, it is possible to evaluate or predict the tendency of the classification related to the hazard of a chemical substance. For example, the standard of the hazard of a chemical substance may change due to external factors such as social factors in addition to the toxicity of the chemical substance itself. Therefore, it is difficult to evaluate the hazard only from the structural characteristics of the chemical substance. The evaluation support apparatus according to the present embodiment classifies a plurality of documents based on their relatedness, and outputs information for evaluating or predicting the tendency of the classification related to the hazard of the chemical substance based on statistical information using the properties of the chemical substance described in the documents. Therefore, the evaluation support apparatus according to the present embodiment can evaluate or predict the tendency of the classification related to the hazard of the chemical substance.

In particular, the evaluation support apparatus according to the present embodiment outputs information for evaluating or predicting the tendency of the classification related to the hazard of the chemical substance based on statistical information using the properties of the chemical substance described in the classified documents. By referring to the statistical information of the classification related to the hazard of a specific chemical substance, it is possible to identify the chemical substance whose hazard is actively discussed or the classification of the hazard that is actively discussed with respect to a specific chemical substance.

Further, the evaluation support apparatus in the present embodiment classifies documents based on at least one of toxicity, bioaccumulation, recalcitrance, regional distribution, flammability, or greenhouse effect of the chemical substance. Because the hazard of a chemical substance is discussed from various viewpoints, it is possible to evaluate the hazard of a chemical substance in detail by simultaneously evaluating various classification tendencies.

Furthermore, the evaluation support apparatus in the present embodiment classifies academic papers based on the citation relationship or the natural language processing result. Although the hazard of a chemical substance is discussed from various viewpoints, it has not been conventionally known that it is possible to classify academic papers based on the citation relationship or the like. By classifying academic papers based on the citation relationship or the natural language processing result, it is expected to obtain highly reliable information for evaluating the hazard of a chemical substance.

Although the embodiment has been described above, it will be understood that various changes in form and details are possible without departing from the spirit and scope of the claims.

The present international application is based upon and claims priority to Japanese patent application no. 2022-86247 filed on May 26, 2022, the entire contents of which are incorporated herein by reference.

Reference Signs List

10 evaluation support apparatus
11 input unit
12 converting unit
13 learning unit
14 classifier storage unit
15 extracting unit
16 output unit

**Claims**

1. An evaluation support apparatus comprising:

   a control unit, wherein
   the control unit outputs information for evaluating or predicting a tendency of classification regarding a hazard of a chemical substance, based on relatedness of a plurality of documents.

2. The evaluation support apparatus according to claim 1, wherein the control unit performs statistical processing of information classifying the plurality of documents for each property of the chemical substance, to thereby output the information for evaluating or predicting the tendency of the classification related to the hazard of the chemical substance.

3. The evaluation support apparatus according to claim 2, wherein the control unit classifies the plurality of documents based on whether or not the property of the chemical substance is described.

4. The evaluation support apparatus according to claim 3, wherein the control unit performs the statistical processing of the information classifying the plurality of documents based on characteristic information of the chemical substance described in the document.

5. The evaluation support apparatus according to claim 4, wherein the control unit evaluates or predicts the tendency of the classification related to the hazard of the chemical substance, from the output information.

6. The evaluation support apparatus according to claim 5, wherein the control unit evaluates or predicts the tendency of the chemical substances having similar characteristic information, from the characteristic information of the chemical substance described in the document.

7. The evaluation support apparatus according to claim 2, wherein the property of the chemical substance includes at least one of toxicity, bioaccumulation, recalcitrance, regional distribution, flammability, or greenhouse effect of the chemical substance.

8. The evaluation support apparatus according to claim 2, wherein the property of the chemical substance includes information that lowers an applicability to the classification related to the hazard of the chemical substance.

9. The evaluation support apparatus according to claim 8, wherein the information lowering the applicability includes at least one of a processing method or a decomposition method of the chemical substance.

10. The evaluation support apparatus according to claim 2, wherein the control unit classifies the plurality of documents based on a distributed expression in which the documents highly related to each other are arranged close to each other.

11. The evaluation support apparatus according to claim 10, wherein the control unit

  attaches, to a document among the plurality of documents, document information indicating whether or not the property of the chemical substance is described, and
  classifies the document in which the property of the chemical substance is described, based on the document information.

12. The evaluation support apparatus according to claim 2, wherein the control unit classifies the plurality of documents based on a citation relationship between the plurality of documents.

13. The evaluation support apparatus according to claim 12, wherein the document is an academic paper.

14. The evaluation support apparatus according to claim 2, wherein the control unit classifies the plurality of documents based on a result of natural language processing of contents described in the document.

15. An evaluation method comprising:
  a procedure of evaluating or predicting a tendency of classification regarding a hazard of a chemical substance, based on relatedness of a plurality of documents.

16. A program that causes a control unit included in an evaluation support apparatus to execute:
  a procedure of outputting information for evaluating or predicting a tendency of classification regarding a hazard of a chemical substance, based on relatedness of a plurality of documents.

# FIG.1

DOCUMENT DATA

ANNOTATION DATA

SEARCH TARGET DATA

EVALUATION SUPPORT APPARATUS 10

DOCUMENT VECTOR

DOCUMENT VECTOR

CLASSIFIER

HAZARD CATEGORY

TOXICITY    BIOACCUMULATION

RECALCITRANCE    REGIONAL DISTRIBUTION

EP 4 535 195 A1

# FIG.2

EVALUATION SUPPORT APPARATUS 10

PROCESSOR 101

MEMORY 102

AUXILIARY STORAGE DEVICE 103

108

OPERATION DEVICE 104

DISPLAY DEVICE 105

COMMUNICATION DEVICE 106

DRIVE DEVICE 107

STORAGE MEDIUM 109

EP 4 535 195 A1

# FIG.3

10

EVALUATION SUPPORT APPARATUS

11

INPUT UNIT

12

CONVERTING UNIT

13

LEARNING UNIT

15

EXTRACTING UNIT

14

CLASSIFIER STORAGE UNIT

16

OUTPUT UNIT

# FIG.4

START

S1
ACQUIRE DOCUMENT DATA RELATING TO SUBSTANCE

S2
GENERATE DOCUMENT VECTOR

S3
ARRANGE IN MULTIDIMENSIONAL SPACE

S4
LEARN BOUNDARY SURFACE OF EACH CATEGORY

S5
EXTRACT PAPER OF EACH CATEGORY

S6
OUTPUT INFORMATION BASED ON CLASSIFICATION RESULT

END

# FIG.5

# FIG.6

# FIG.7

| TOXICITY | |
|---|---|
| NAME OF SUBSTANCE | NUMBER OF PAPERS |
| A | 18 |
| B | 12 |
| C | 6 |
| . . . | . . . |

## INTERNATIONAL SEARCH REPORT

International application No.

**PCT/JP2023/018957**

### A. CLASSIFICATION OF SUBJECT MATTER

*G06F 16/906*(2019.01)i; *G06Q 10/04*(2023.01)i
FI:  G06F16/906; G06Q10/04

According to International Patent Classification (IPC) or to both national classification and IPC

### B. FIELDS SEARCHED

Minimum documentation searched (classification system followed by classification symbols)

G06F16/906; G06Q10/04

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | CN 111651605 A (UNIVERSITY OF ELECTRONIC SCIENCE AND TECHNOLOGY OF CHINA) 11 September 2020 (2020-09-11)<br>paragraphs [0007]-[0027] | 1-16 |
| A | JP 11-015835 A (FUJI XEROX CO LTD) 22 January 1999 (1999-01-22)<br>paragraphs [0001], [0013]-[0014], [0023], [0037]-[0038], fig. 8 | 1-16 |
| A | JP 2001-092825 A (NEC CORP) 06 April 2001 (2001-04-06)<br>paragraphs [0001], [0098]-[0102], fig. 1 | 1-16 |
| A | US 2011/0302171 A1 (WALDO PATRICK BLACKMON) 08 December 2011 (2011-12-08)<br>paragraphs [0001], [0057], [0059], [0090], fig. 24 | 1-16 |
| A | JP 2007-153767 A (UNIV TOKUSHIMA) 21 June 2007 (2007-06-21)<br>paragraphs [0013]-[0014] | 1-16 |

☐ Further documents are listed in the continuation of Box C.   ☑ See patent family annex.

| | |
|---|---|
| *  Special categories of cited documents:<br>"A"  document defining the general state of the art which is not considered to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
|---|---|
| **13 July 2023** | **25 July 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
**Information on patent family members**

International application No.

**PCT/JP2023/018957**

| Patent document cited in search report | | | Publication date (day/month/year) | Patent family member(s) | | | Publication date (day/month/year) |
|---|---|---|---|---|---|---|---|
| CN | 111651605 | A | 11 September 2020 | (Family: none) | | | |
| JP | 11-015835 | A | 22 January 1999 | (Family: none) | | | |
| JP | 2001-092825 | A | 06 April 2001 | US<br>column 1, lines 43-46, column 3, line 52 to column 4, line 14, fig. 5<br>KR 10-2001-0067187<br>CN | 6738761<br><br><br><br>1289097 | B1<br><br><br><br>A<br>A | |
| US | 2011/0302171 | A1 | 08 December 2011 | WO<br>EP | 2012/174219<br>2721545 | A1<br>A1 | |
| JP | 2007-153767 | A | 21 June 2007 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018049376 A

- JP 2022086247 A

**Non-patent literature cited in the description**

- **TANG, J.** ; **QU, M.** ; **WANG, M.** ; **ZHANG, M.** ; **YAN, J.** ; **MEI, Q**. Line: Large-scale information network embedding. *Proceedings of the 24th international conference on world wide web*, 2015, 1067-1077

- **BELTAGY, I.** ; **LO, K.** ; **COHAN, A.** SciBERT: A pretrained language model for scientific text. *arXiv:1903.10676*, 2019